Europäisches Patentamt

European Patent Office

Office européen des brevets

(11) Publication number: **0 334 567**
**A2**

# EUROPEAN PATENT APPLICATION

(21) Application number: 89302694.8

(51) Int. Cl.⁴: **A61F 2/06**

(22) Date of filing: 17.03.89

(30) Priority: 21.03.88 GB 8806632
12.10.88 GB 8823891

(43) Date of publication of application:
27.09.89 Bulletin 89/39

(84) Designated Contracting States:
DE FR GB IT

(71) Applicant: ETHICON INC.
U.S. Route 22
Somerville New Jersey 08876(US)

Applicant: THE UNIVERSITY OF LIVERPOOL
Mount Pleasant PO BOX 147
Liverpool L69 3BX(GB)

(72) Inventor: Annis, David
Little Hey Dibbinsdale Road
Bromborough Merseyside L63 0AU(GB)
Inventor: Berry, John Phillip
5 Victoria Drive West Kirby
Wirral Merseyside L48 0QU(GB)

(74) Representative: Howick, Nicholas Keith et al
CARPMAELS & RANSFORD 43 Bloomsbury
Square
London WC1A 2RA(GB)

(54) Improvements in synthetic vascular grafts.

(57) A synthetic vascular graft (10) has a fibrous structure (11) and mechanical properties matching substantially mechanical properties of its natural counterpart, the fibrous structure including coating means of non-biodegradable elastomeric material for protecting fibres of the structure from tissue and capillary ingrowth into the wall of the graft, the coating (12) being of a material and thickness not to alter adversely the mechanical properties of the graft before the coating (12) is present. Alternatively or additionally, individual fibres within the structure are coated.

Fig. 1

EP 0 334 567 A2

# IMPROVEMENTS IN SYNTHETIC VASCULAR GRAFTS

The invention relates to synthetic vascular grafts, and more particularly but not exclusively to synthetic vascular grafts of electrostatically spun polymeric material.

There have been many disclosures of electrostatically spun synthetic vascular grafts made from a fibreizable liquid, such as polyurethane in solution. Examples of such disclosures are Annis et al (trans. Am. Soc. Intern. Organs), European Patent Application No. 0009941, and British Patent Application Nos. 2121286A, 2120946A and 2142870A. The electrostatic spinning method has been consistently developed and particularly useful structures have been obtained from the point of view of mechanical, physical and compatability characteristics as substitutes for their natural counterparts. All such grafts will be referred to herein as "grafts of the type defined".

According to the invention, there is provided a synthetic vascular graft having a fibrous structure and mechanical properties matching substantially mechanical properties of its natural counterpart, the fibrous structure including coating means of non-biodegradable elastomeric material for protecting fibres of the structure from tissue and capillary ingrowth into the wall of the graft, whereby vulnerability of the structure to attack by phagocytic cells is reduced, the coating means being of a material and thickness not to alter adversely the mechanical properties of the graft before the coating means is present.

The graft before application of the coating means is preferably an electrostatically spun polymeric graft, and may advantageously be a graft as disclosed in EP-A-223374A or EP-A-266035A.

The coating means preferably includes an external coating of the graft to present an impervious covering against tissue and capillary ingrowth. The coating means may comprise a coating of individual fibres of the fibrous structure throughout the structure.

The coating means may comprise a combination of individual fibre coatings and an external, impervious coating.

The non-biodegradable material may be silicone rubber, for example that sold by Dow Corning under their Trade Mark SILASTIC. This material is available in approved medical grade. There are alternative materials which may be used, subject to medical clearance, for example polyisobutylene.

The external impervious coating may be between 40μm and 100μm, although this figure may represent an average thickness where the external, uncoated graft surface undulates. For example, the uncoated external surface of a graft as disclosed in EP-A-266035A may undulate by 50 to 100μm between peaks and troughs.

The coating means may be applied from a solution of 40% SILASTIC (Trade Mark) weight for weight in 60% hexane for the external impervious layer and 2.5% SILASTIC (Trade Mark) weight for weight in 97.5% hexane for individual fibre coatings.

The external layer may be applied by dipping, painting, spraying or electrostatic spinning.

The invention further provides a method of providing an impervious outer layer of non-biodegradable elastomeric material on an electrostatically spun structure comprising the steps of forming a solution of said non-biodegradable material, dipping said electrostatically spun structure mounted on a former into said solution, and drying the coated structure.

Where the material is silicone rubber, the method includes a curing step after drying.

During the drying step the structure is preferably rotated slowly (for example at 1 revolution per second) to provide evenness of the outer layer.

The solution may be 40% of Dow Corning SILASTIC (Trade Mark) to 60% hexane, but these proportions may be varied.

Curing may take place at 75°C for ½ hour but these figures may be altered.

The invention further provides a method of coating individual fibres throughout the structure comprising the steps of submerging a graft in a solution containing elastomeric material at a strength to enable full passage of the solution through the structure, applying a vacuum to a vessel containing the submerged structure, releasing the vacuum, removing the graft and drying the graft.

By way of example, embodiments of synthetic vascular grafts according to the invention and methods of providing coatings will now be described with reference to the accompanying drawings, in which:-

Figure 1 is a sectional view through an electrostatically spun structure according to the invention;

Figure 2 is a diagrammatic representation of the sequence of production of an embodiment of a structure according to the invention;

Figure 3 is a side view of a bent graft to illustrate kinking characteristics;

Figure 4 is a schematic view of a coated graft;

Figure 5 is a sectional photographic view of a coated graft magnified 110 times;

Figure 6 is a sectional photographic view of a coated graft magnified 54 times;

Figure 7 is a photographic view magnified 114 times of the outer surface of a coated graft;

Figure 8 is a results table showing physical characteristics;

Figure 9 is a photographic microscopic view magnified 5300 times of a fibrous structure of an uncoated graft; and

Figure 10 is a photographic microscopic view magnified 5300 times of a fibrous structure with coated fibres.

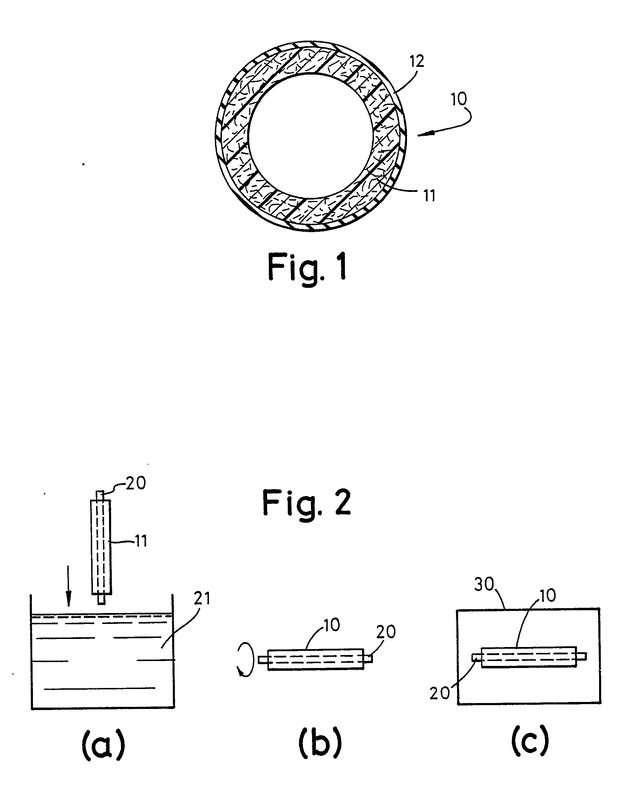

Figure 1 is a sectional view through a tubular synthetic vascular graft generally indicated at 10. The graft includes an inner tubular portion 11 of electrostatically spun fibres of a material such as polyurethane. The electrostatically spun structure may take a variety of different forms but a preferred form may be the structure described in EP-A-223374A or EP-A-266035A and may include fibres of different diameters and may include voids. The structure may include an impermeable layer within the structure. Surrounding the electrostatically spun structure 11 is an outer layer 12 of non-biodegradable material, the layer 12 being impervious such that, in use in a body, living tissue outside the vascular graft 10 has no access to material of the electrostatically spun structure 11. The material 12 in this embodiment is of silicone rubber (sold by Dow Corning under their Trade Mark SILASTIC) of approved medical grade. It will be appreciated that othere non-biodegradable material may be used provided it has suitable medical clearance, an alternative being polyisobutylene. Other materials may also be suitable. The layer 12 is between 40 and 100 microns thick in this embodiment.

Figure 2 shows diagrammatically manufacturing stages for provision of the layer 12. Figure 2(a) shows a mandrel 20 carrying the electrostatically spun, tubular structure 11 thereof, about to be dipped in a solution 21 of silicone rubber. In this particular embodiment, the solution is 40%Dow Corning SILASTIC (Trade Mark) medical grade in 60% hexane which has been found to give suitable viscosity for this operation. The viscosity may be varied, however, depending on circumstances. In the dipping and removal operation, a coating of silicone rubber in solution is retained on the outer surface of the structure 11. It is important to note that a solvent is used which does not affect the material of the fibrous structure 11, commonly polyurethane. Hexane is suitable for this purpose but other solvents could be used.

Figure 2(b) shows a drying operation where the mandrel 20 now carrying the graft 10 including the outer layer is dried and rotated at 1 revolution per second for approximately 15 minutes until dry.

Figure 2(c) illustrates diagrammatically curing of the outer layer of silicone rubber by placing the product in a vacuum oven for $\frac{1}{2}$ hour at 75°C.

After the curing process, the product is removed from the mandrel 20 and is ready to use.

Silicone rubber has similar elastomeric properties to a synthetic vascular graft formed from electrostatically spun polyurethane, which properties are compatible with their natural counterparts. Indeed the provision of an outer layer of silicone rubber has been found to give a smoother outer profile than the electrostatically spun fibrous structure itself, with less irregularities. Provision of the outer layer of silicone rubber adds to the weight of the graft which may make handling easier. The product with the outer layer of silicone rubber is also easier to mark and may improve twisting resistance characteristics and kinking resistance.

The presence of the outer layer of non-biodegradable material ensures that fibres of the electrostatically spun structure are protected from contact with living bodily tissue while leaving the electrostatically spun fibrous structure open to contact on the inside thereof with blood and, at its extremities, with natural arteries or veins. Attractive properties of electrostatically spun synthetic grafts are thus retained with the added attractiveness of protection of the electrostatically spun material from material outside the graft.

In the following description, a graft as described in EP-A-266035A having fibres of varying sizes and a meld layer is referred to as a MELF graft.

An external coating to a MELF graft is applied by the following method:-

a) the graft, still on the mandrel, is sealed at one end with a cable tie, to prevent the SILASTIC from being forced up between the graft and the mandrel.

b) the graft is dipped vertically into 40% SILASTIC/hexane solution and slowly withdrawn.

c) it is turned round 90 degrees so that the graft is horizontal, and rotated about the longitudinal axis of the mandrel on a machine at approximately 60 revolutions per minute.

The hexane evaporates after about fifteen minutes, leaving a thin coat of uncured SILASTIC on the surface of the graft. This is moved to a warm, humid environment to cure the SILASTIC.

The horizontal rotation of the graft while the solution is still liquid allows the SILASTIC to concentrate, or fill up, in the valleys of the graft. The cured coating is found to be slightly thicker in these regions when

3

examined in cross-section.

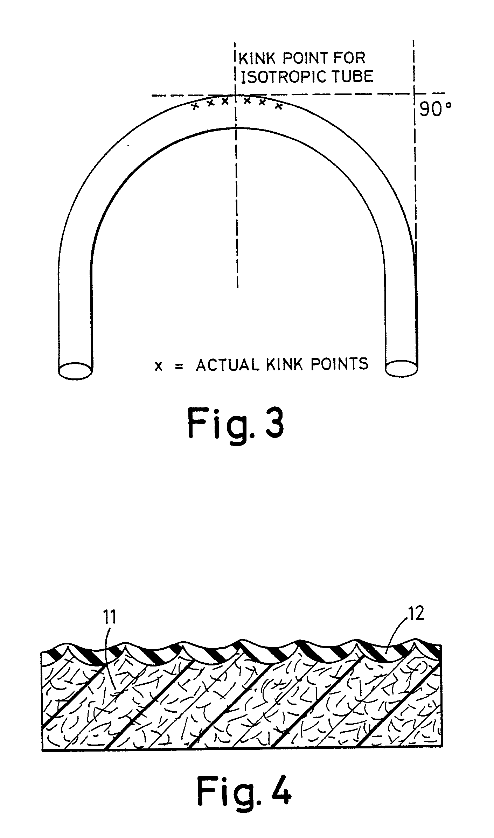

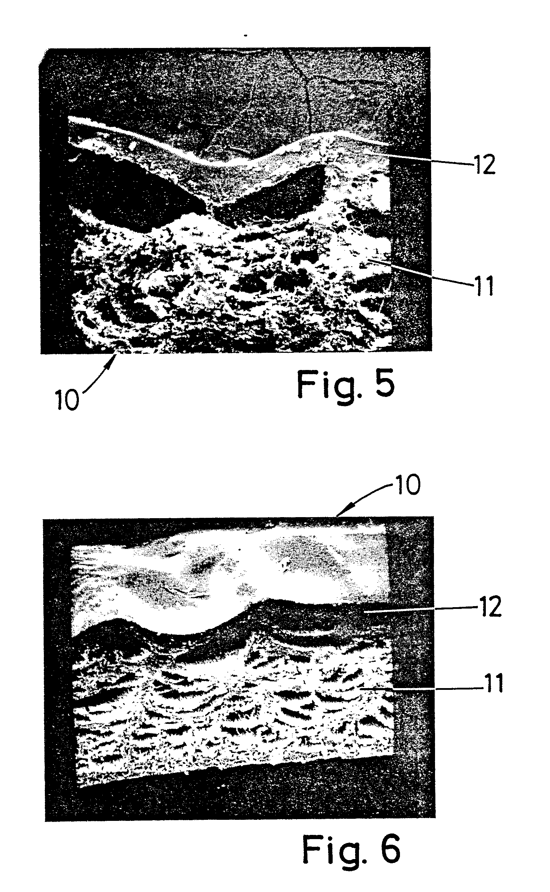

This enhances the mechanical properties of the graft in the following way. When a MELF graft is bent gradually into a progressively tightening arc of a circle, up to the point at which it kinks, the actual point of kink is not usually at the centre of the arc, where an isotropic tube (eg an ordinary rubber tube) would kink but can be several millimetres away from this point (Figure 3). This is because peaks and valleys on the grafts are not regularly spaced, nor of an equal depth, and the graft will kink at one of these preferred points of weakness. With the horizontal drying technique, the deeper valles are partially filled, which reduces the effect of the weak spots and enhances the favourable bending characteristics of the -graft (Figure 4).

Secondly, the SILASTIC coating improves the self-sealing properties of the graft after it has been punctured for dialysis with 4mm diameter needles. A semi-circular flap is cut out by the needle and this springs back into place, owing to the elastomeric nature of the material. This will help prevent perigraft haematomas and infection at the needle puncture site.

It will be appreciated that external coating can be achieved by spraying, painting or electrostatic spinning, instead of dipping.

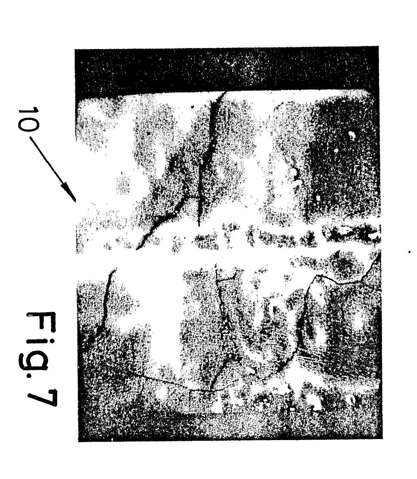

Figures 5, 6 and 7 illustrate the contours of a coated graft, the elastomeric coating being indicated by numeral 110.

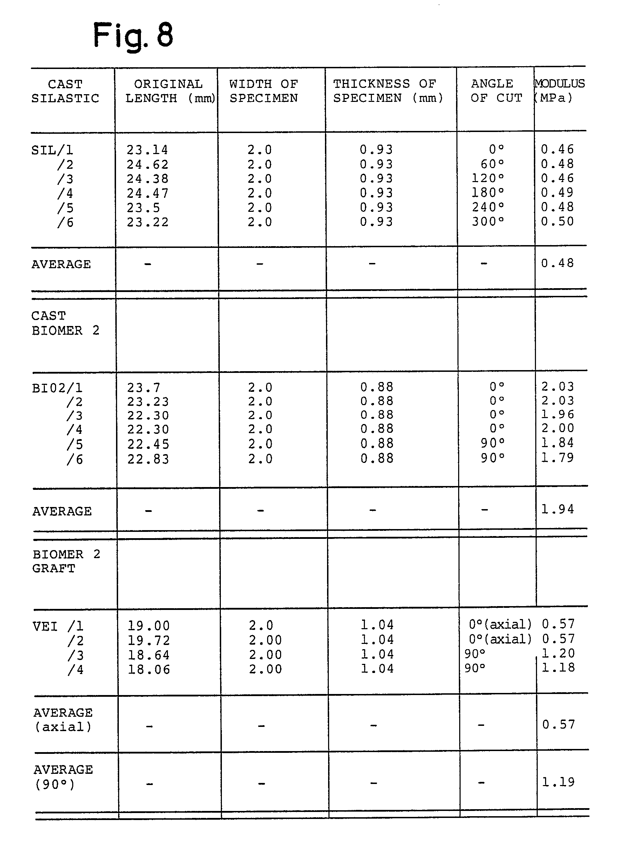

Figure 8 is a table illustrating physical characteristics of SILASTIC compared to a chosen polymer referred to as BIOMER 2. The low modulus of SILASTIC compared to BIOMER 2 is to be noted.

Turning to the coating of individual fibres within the graft, the treatment method is different from the sheathing of the graft with a $40\mu m$ to $100\mu m$ coat of SILASTIC. Both individual fibre coating and sheathing may be employed, since they address different problems. The former could prevent chemical degradation of fibres, protect the innner layer of a graft and cut ends where of necessity there is a break in the external sheath, whereas the latter is to prevent tissue and capillary ingrowth and subsequent action by hostile cells.

Method of coating each individual Biomer fibre with a thin layer of SILASTIC rubber.

Method

A two and a half percent w/w solution of Dow Corning SILASTIC in hexane is made. The graft is submerged in the solution and the vessel is placed in a vacuum chamber. The chamber is evacuated to 760mm Hg. and held for approximately two minutes. The vacuum is released and the graft is hung up to dry in a chamber at 40° and at 40% relative humidity for approximately one hour. This process is repeated.

Evidence of SILASTIC coating.

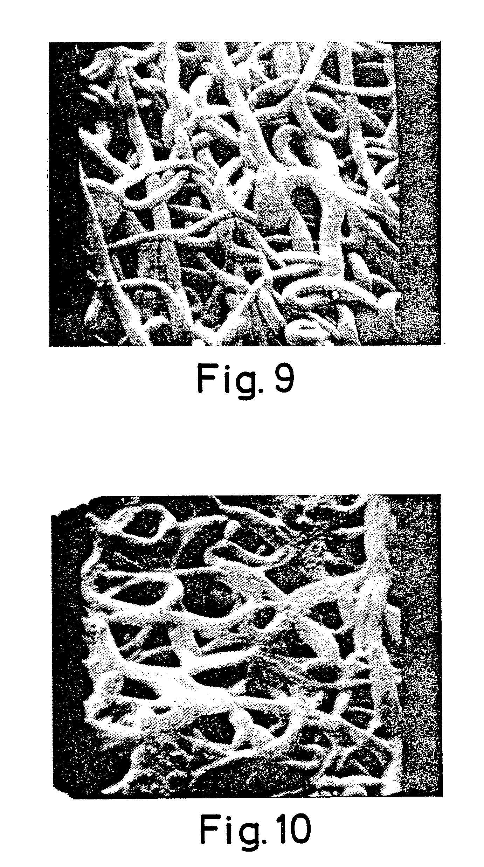

1) The SEM photograph (Figure 10) of a coated graft compared to the SEM photograph (Figure 9) of an uncoated graft shows that there is a webbing of presumably SILASTIC at the crossover points of the fibres. (two dips at 2.5%)

2) The graft increases in weight compared with the undipped weight each time it is dipped in the 2.5% solution and dried.

| 1 dip | weight increase | 5.9% |
|---|---|---|
| 2 dips | weight increase | 10.8% |
| 3 dips | weight increase | 16.1% |
| 4 dips | weight increase | 19.2% |

3) It has been calculated that, for 2.5% dips, the fibres have a coating of SILASTIC that is about $0.2\mu m$ thick.

A graft according to this embodiment of the invention coated both externally and with individual fibre coatings has the advantage that, while retaining physically advantageous characteristics, problems of degradation from outside, inside and at cut ends are overcome.

Claims

1. A synthetic vascular graft having a fibrous structure and mechanical properties matching substantially mechanical properties of its natural counterpart, the fibrous structure including coating means of non-biodegradable elastomeric material for protecting fibres of the structure from tissue and capillary ingrowth into the wall of the graft, the coating means being of a material and thickness not to alter adversely the mechanical properties of the graft before the coating means is present.

2. A synthetic vascular graft as claimed in Claim 1 wherein the graft before application of the coating means is an electrostatically spun polymeric graft.

3. A synthetic vascular graft as claimed in Claim 1 or Claim 2 wherein the coating means includes an external coating of the graft to present an impervious covering against tissue and capillary ingrowth.

4. A synthetic vascular graft as claimed in Claim 3 wherein the external impervious coating is of a thickness between 40μm and 100μm.

5. A synthetic vascular graft as claimed in Claim 3 or Claim 4 wherein the external impervious coating is applied from a solution of 40% SILASTIC (Trade Mark) weight for weight in 60% hexane.

6. A synthetic vascular graft as claimed in any one of Claims 3 to 5 wherein the external impervious coating is applied by dipping the uncoated graft in a solution of the elastomeric material.

7. A synthetic vascular graft as claimed in any one of Claims 3 to 5 wherein the external impervious coating is applied by painting the elastomeric material on the uncoated graft.

8. A synthetic vascular graft as claimed in any one Claims 3 to 5 wherein the external impervious coating is applied by spraying to the uncoated graft.

9. A synthetic vascular graft as claimed in any one of Claims 3 to 5 wherein the external impervious coating is applied by electrostatically spinning the elastomeric material to the graft without the coating.

10. A synthetic vascular graft as claimed in any one Claims 1 to 9 wherein the coating means comprise a coating of individual fibres of the fibrous structure throughout the structure.

11. A synthetic vascular graft as claimed in Claim 10 wherein the individual fibre coatings are achieved by application of a solution of 2.5% SILASTIC (Trade Mark) weight for weight in 97.5% hexane.

12. A synthetic vascular graft as claimed in Claim 10 or Claim 11 wherein the individual fibre coatings are applied by dipping the uncoated graft in a solution of the elastomeric material.

13. A method of providing an impervious outer layer of non-biodegradable elastomeric material on an electrostatically spun structure comprising the steps of forming a solution of said non-biodegradable material, dipping said electrostatically spun structure mounted on a former into said solution, and drying the coated structure.

14. A method as claimed in Claim 13 including a curing step after drying where the material is silicone rubber.

15. A method as claimed in Claim 14 wherein curing takes place at 75°C for ½ hour.

16. A method as claimed in any one of Claims 13 to 15 wherein the structure is rotated slowly during the drying step to provide evenness of the outer layer.

17. A method as claimed in any one of Claims 13 to 16 wherein the solution is of 40% SILASTIC (Trade Mark) weight for weight in 60% hexane.

18. A method of coating individual fibres throughout the structure of a synthetic vascular graft having a fibrous structure, which method comprises the steps of submerging the graft in a solution containing elastomeric material at a strength to enable full passage of the solution through the structure, applying a vacuum to a vessel containing the submerged structure, releasing the vacuum, removing the graft and drying the graft.

19. A method as claimed in Claim 18 wherein the solution is 2.5% SILASTIC (Trade Mark) weight for weight in 97.5% hexane.

20. A method as claimed in Claim 18 or Claim 19 wherein the drying step involves placing the graft in a chamber at 40°C and 40% relative humidity for approximately one hour.

21. A method as claimed in any one of Claims 18 to 20 wherein the method steps are repeated to produce the final graft.

22. A method of coating a synthetic vascular graft having a fibrous structure, the coating being both of individual fibres and an external impervious coating, which method comprises the combination of a method as claimed in any one of Claims 18 to 21 followed by a method as claimed in any one of Claims 13 to 17.

Fig. 1

Fig. 2

(a)          (b)          (c)

KINK POINT FOR
ISOTROPIC TUBE

90°

x = ACTUAL KINK POINTS

## Fig. 3

11    12

## Fig. 4

Fig. 5

Fig. 6

Fig. 7

# Fig. 8

| CAST SILASTIC | ORIGINAL LENGTH (mm) | WIDTH OF SPECIMEN | THICKNESS OF SPECIMEN (mm) | ANGLE OF CUT | MODULUS (MPa) |
|---|---|---|---|---|---|
| SIL/1 | 23.14 | 2.0 | 0.93 | 0° | 0.46 |
| /2 | 24.62 | 2.0 | 0.93 | 60° | 0.48 |
| /3 | 24.38 | 2.0 | 0.93 | 120° | 0.46 |
| /4 | 24.47 | 2.0 | 0.93 | 180° | 0.49 |
| /5 | 23.5 | 2.0 | 0.93 | 240° | 0.48 |
| /6 | 23.22 | 2.0 | 0.93 | 300° | 0.50 |
| AVERAGE | – | – | – | – | 0.48 |
| CAST BIOMER 2 | | | | | |
| BIO2/1 | 23.7 | 2.0 | 0.88 | 0° | 2.03 |
| /2 | 23.23 | 2.0 | 0.88 | 0° | 2.03 |
| /3 | 22.30 | 2.0 | 0.88 | 0° | 1.96 |
| /4 | 22.30 | 2.0 | 0.88 | 0° | 2.00 |
| /5 | 22.45 | 2.0 | 0.88 | 90° | 1.84 |
| /6 | 22.83 | 2.0 | 0.88 | 90° | 1.79 |
| AVERAGE | – | – | – | – | 1.94 |
| BIOMER 2 GRAFT | | | | | |
| VEI /1 | 19.00 | 2.0 | 1.04 | 0°(axial) | 0.57 |
| /2 | 19.72 | 2.00 | 1.04 | 0°(axial) | 0.57 |
| /3 | 18.64 | 2.00 | 1.04 | 90° | 1.20 |
| /4 | 18.06 | 2.00 | 1.04 | 90° | 1.18 |
| AVERAGE (axial) | – | – | – | – | 0.57 |
| AVERAGE (90°) | – | – | – | – | 1.19 |

Fig. 9

Fig. 10